# NEUE EUROPÄISCHE PATENTSCHRIFT

(11) **EP 1 996 028 B2**
(45) Veröffentlichungstag und Bekanntmachung des Hinweises auf die Entscheidung über den Einspruch: **02.12.2015**
(45) Hinweis auf die Patenterteilung: 05.01.2011
(21) Anmeldenummer: 07726771.4
(22) Anmeldetag: 09.03.2007
(51) Int. Cl.: A23K 1/165, C12N 9/96, C12N 9/98

(54) **FESTE ENYMFORMULIERUNGEN UND VERFAHREN ZU DEREN HERSTELLUNG**
SOLID ENZYME FORMULATIONS AND PROCESS FOR THEIR PREPARATION
FORMULATIONS ENZYMATIQUES SOLIDES ET LEURS PROCÉDÉS DE PRÉPARATION

(30) Priorität: 10.03.2006 EP 06004998
(43) Veröffentlichungstag der Anmeldung: 03.12.2008
(73) Patentinhaber: BASF SE, 67056 Ludwigshafen (DE)
(72) Erfinder: LOHSCHEIDT, Markus, 69121 Heidelberg (DE); BETZ, Roland, 67150 Niederkirchen (DE); BRAUN, Jörg, 76879 Essingen (DE); PELLETIER, Wolf, 76879 Ottersheim (DE)
(86) Internationale Anmeldenummer: PCT/EP2007/052256
(87) Internationale Veröffentlichungsnummer: WO 2007/104725

(56) Entgegenhaltungen:
- EP-A1- 0 758 018
- EP-A2- 0 257 996
- US-A- 5 972 669
- US-B1- 6 610 519

## Beschreibung

Die vorliegende Erfindung betrifft neuartige feste Enzymformulierungen, umfassend Abmischungen aus wenigstens einer salzstabilisierten Enzymzusammensetzung, wenigstens einem partikelförmigen Träger und wenigstens einer hydrophoben Flüssigkeit. Weiterhin betrifft die Erfindung Verfahren zur Herstellung solcher fester Enzymformulierungen sowie Verfahren zur Herstellung von Tierfutter Nahrungsmittel und Nahrungsergänzungsmittel, welche derartige Enzymformulierungen enthalten.

### Hintergrund der Erfindung

Aus dem Stand der Technik sind zahlreiche feste Enzymzusammensetzungen bekannt, welche beispielsweise durch Sprühtrocknung flüssiger Enzymlösungen hergestellt werden. Es ist weiterhin bekannt, dass die Enzymstabilität bei derartigen Sprühtrocknungsprozessen durch Zugabe stabilisierender Salze, wie beispielsweise Magnesiumsulfat, signifikant erhöht werden kann. Man erhält daher auf diese Weise feste Enzymzusammensetzungen, welche auch nach dem Sprühtrocknen eine hohe prozentuale Enzymaktivität aufweisen. Beispielsweise werden in der EP-A-0 758 018 lagerstabile und verarbeitungsstabile feste Enzymzusammensetzungen beschrieben, welche man durch Trocknung einer Lösung, enthaltend mindestens ein Enzym und ein wasserlösliches anorganisches Salz, erhält. Die dort beschriebenen Enzymzusammensetzungen finden vorzugsweise Anwendung als Zusatz für feste Tierfutterzusammensetzungen.

Aus der US 5,972,669 sind feste, salzstabilisierte Enzymlösungen bekannt, die in Futtermitteln verwendet werden können, wobei diese im Gemisch mit anderen Futtermittelkomponenten, wie Mehlen und Sojaöl, vorliegen können.

Aus der US 6,610,519 sind feste Phytasegranulate bekannt, welche mittels Milchsäure (aus Com Steep Liquor) stabilisiert sind und gegebenenfalls ein Fettcoating aufweisen.

Für die Herstellung solcher Enzym-additivierter Tierfutterzusammensetzungen ist es wünschenswert, dass das Enzym möglichst gleichmäßig verteilt in dem fertigen Futterpräparat vorliegt. Da die trockenen Enzympräparate das Enzym in hoher Konzentration enthalten, ist ein Zusatz von deutlich weniger als 1 Gew.-%, bezogen auf das Gesamtgewicht der Futterzusammensetzung meist völlig ausreichend, um die gewünschte Enzymaktivität für die Futterzusammensetzung bereitzustellen. Je geringer die erforderliche Menge an zu dosierendem Enzym ist, desto schwieriger ist es aber, eine gleichmäßige Verteilung der Enzymaktivität im fertigen Futtermittelpräparat zu erreichen. Die gleiche Schwierigkeit beobachtet man natürlich auch bei der Herstellung von

Nahrungs- und Nahrungsergänzungsmitteln, denen hochkonzentrierte feste Enzymzusammensetzungen möglichst gleichmäßig verteilt zuzusetzen sind.

Es besteht daher die Aufgabe der Erfindung, einen Weg zu finden, der es ermöglicht, hochkonzentrierte feste Enzymzusammensetzungen, welche im wesentlichen nur Enzym und stabilisierenden Träger enthalten, in eine Form zu bringen, welche eine gleichmäßige und reproduzierbare Zudosierung zu Nahrungs- und Futtermitteln gewährleistet. Gleichzeitig sollte auch gewährleistet sein, dass die dazu verwendeten Formulierungen gute Verarbeitungseigenschaften, wie verringerte Staubneigung, gutes Fließverhalten und enge Partikelgrößenverteilung besitzen.

### Zusammenfassung der Erfindung

Obige Aufgabe konnte überraschenderweise durch Bereitstellung einer festen Enzymformulierung nach Anspruch 1 gelöst werden, welche man durch Mischen einer partikelförmigen, salzstabilisierten Enzymzusammensetzung, eines partikelförmigen Trägers sowie einer hydrophoben Flüssigkeit erhält. Insbesondere war überraschend, dass die erfindungsgemäß hergestellten festen Formulierungen besonders gut handhabbar sind, da sie eine hohe Entmischungsstabilität, äußerst geringe Staubneigung und, trotz Zugabe von hydrophober Flüssigkeit, ein ausgezeichnetes Fließverhalten zeigen.

### Figurenbeschreibung

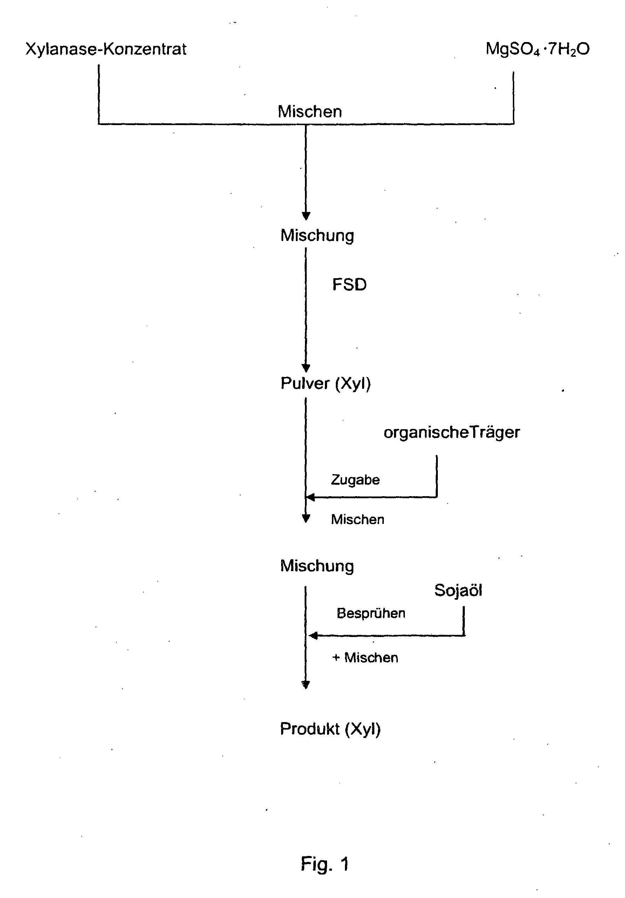

Figur 1 veranschaulicht anhand eines Fließschemas eine bevorzugte Ausführungsform der vorliegenden Erfindung, insbesondere die Herstellung einer festen Xylanase-Formulierung. Dazu wird ein Xylanase-haltiges Flüssigkonzentrat mit Magnesiumsulfat vermischt, in einer Sprühvorrichtung zu einem Xylanase-haltigen, stabilisierten Pulver getrocknet und gleichzeitig agglomeriert, wobei man z.B. Partikel mit einer Größe im Bereich von 50 bis 250 µm erhalten kann. In einem nächsten Schritt wird das Xylanase-haltige Trockenpulver mit einem festen organischen Träger vermischt und gleichzeitig oder anschließend mit Sojaöl besprüht. Man erhält auf diese Weise eine Xylanasehaltige Formulierung mit geringer Staubneigung und hoher Entmischungsstabilität.

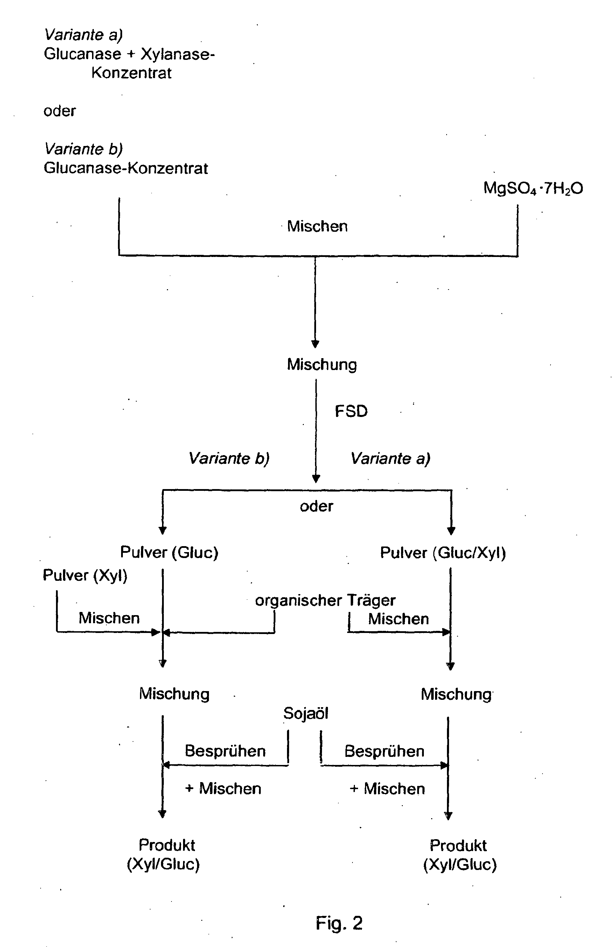

Figur 2 veranschaulicht die Herstellung weiterer erfindungsgemäß bevorzugten festen Enzymformulierungen, welche in unterschiedlichen Ausgestaltungen ein Gemisch aus Xylanase und Glucanase enthalten. Nach Verfahrensvariante (a) geht man von einem flüssigen Mischkonzentrat aus Glucanase und Xylanase aus, wohingegen man bei Verfahrensvariante (b) zunächst von einem flüssigen Glucanase-Konzentrat ausgeht. Nach Verfahrensvariante (a) wird das Mischkonzentrat aus Glucanase und Xylanase wie oben für Figur 1 beschrieben, getrocknet und mit einem organischen Träger vermischt und mit Sojaöl besprüht. Gemäß Verfahrensvariante (b) wird dagegen zunächst ein flüssiges Glucanase-Konzentrat in analoger Weise wie oben bereits für das Xylanase-Konzentrat beschrieben, zu einem Glucanase-haltigen Pulver verarbeitet. Dieses Pulver wird mit einem gemäß Figur 1 hergestellten Xylanasepulver vermischt. Gleichzeitig erfolgt ein Vermischen mit dem organischen Träger und ein Besprühen mit Sojaöl, wobei man ebenfalls eine Xylanase und Glucanase enthaltende Enzymformulierung erhält. Auch die hierbei anfallenden, Glucanase und Xylanase enthaltenden festen Enzymformulierungen zeichnen sich durch geringe Staubneigung und hohe Entmischungsstabilität aus.

### Detaillierte Beschreibung der Erfindung

### a) Bevorzugte Ausführungsformen der Erfindung

Gegenstand der Erfindung sind feste Enzymformulierungen, nach Ansprunch 1 unfassend eine Mischung von a) wenigstens einer partikelförmigen, gekörnten Enzymzusammensetzung von wenigstens einem Enzym und wenigstens einem organischen oder anorganischen Salz eines ein- oder zweiwertigen Metall-Kations mit b) wenigstens einem partikelförmigen anorganischen oder organischen, physiologisch verträglichen Träger und c) wenigstens einer hydrophoben Flüssigkeit mit klebenden Eigenschaften, und insbesondere einer hydrophoben Flüssigkeit mit einem Schmelzpunkt im Bereich von -60 °C bis 30°C, insbesondere von -50 bis 0°C, wie z.B. von -40 bis -5°C oder von -30 bis -10°C;
wobei das Verhältnis der mittleren Partikeldurchmesser von Träger zu Enzymzusammensetzung im Bereich von etwa 1 bis 8 liegt und wobei das Mischungsverhältnis von Enzymzusammensetzung und Träger im Bereich von etwa 1:1000 bis 1:5 Gewichtsteilen liegt.

Gegenstand der Erfindung sind insbesondere Enzymformulierungen der oben bezeichneten Art, umfassend
a) eine partikelförmige Enzymzusammensetzung, enthaltend ein Enzym im Gemisch mit wenigstens einem organischen oder anorganischen Salz eines ein- oder zweiwertigen Kations; oder
b) eine partikelförmige Enzymzusammensetzung, enthaltend mindestens zwei voneinander verschiedene Enzyme im Gemisch mit wenigstens einem organischen oder anorganischen Salz eines ein- oder zweiwertigen Kations; oder
c) mindestens zwei voneinander verschiedene partikelförmige Enzymzusammensetzungen, wobei sich beide Zusammensetzungen dadurch unterscheiden, dass sie mindestens ein unterschiedliches Enzym enthalten, wobei die Enzyme in jeder Zusammensetzung im Gemisch mit wenigstens einem organischen oder anorganischen Salz eines ein- oder zweiwertigen Kations vorliegen.

Insbesondere betrifft die Erfindung Enzymformulierungen, wobei das Verhältnis der mittleren Partikeldurchmesser von Träger zu Enzymzusammensetzung im Bereich von etwa 1 bis 4 oder 1 bis 2 oder 1 bis 1,5 liegt. Dabei liegt die mittlere Partikelgröße von eingesetzter Enzymzusammensetzung und eingesetztem Träger im Bereich von 50 bis 500 µm oder 150 bis 350 µm. Zweckmäßigerweise wird das Mischungsverhältnis von Enzymzusammensetzung und Träger im Bereich von etwa 1:500 bis 1:10 oder 1:100 bis 1:20 Gewichtsteilen eingestellt.

Der Anteil der hydrophoben Flüssigkeit beträgt 0,1 bis 5, 0,2 bis 2, 0,3 bis 1,5 oder 0,3 bis 0,7 Gew.-%, bezogen auf das Gesamtgewicht der Enzymformulierung.

In den erfindungsgemäß verwendeten Enzymzusammensetzungen liegt der Salzanteil im Bereich von 1 bis 30 Gew.-%, 5 bis 25 Gew.-% oder 10 bis 20 Gew.-%, bezogen auf das Gesamtgewicht der Enzymzusammensetzung.

Der prozentuale Anteil an Enzym-Protein in der Enzymzusammensetzung beträgt etwa 0,01 bis 99 Gew.-%, wie z.B. 0,01 bis 80 Gew.-% ,10 bis 80 Gew.-%, 20 bis 75 Gew.-% oder 30 bis 60 Gew.-%.

Neben mindestens einem Enzym und mindestens einem Salz kann die Enzymzusammensetzung darüber hinaus weitere Bestandteile enthalten. Diese können als Bindemittel (z.B. Polymere oder Zucker), als Füllstoff (z.B. Kalk, Lehm, Kohlehydrate, Zucker, Stärke), als Farbstoff oder weiterer Stabilisator dienen. Derartige weitere Bestandteile sind an sich aus dem Stand der Technik bekannt und dem Fachmann geläufig.

Die Restfeuchte der Enzymmischung liegt erfindungsgemäß in einem Bereich von 5 bis 30 Gew.-%, wie z.B. von 5 bis 20 Gew.-%, oder von 7 bis 16 Gew.-%.

Die Erfindung ist auf keine bestimmten Enzyme beschränkt. Insbesondere sind die verwendbaren Enzyme aber ausgewählt unter Hydrolasen (EC 3.), insbesondere Glycosidasen (EC 3.2.1), Peptidasen (EC 3.4) und vor allem Xylanasen, Glucanasen (Hemicellulasen), Cellulasen, Proteasen, Keratinasen, Amylasen, Peptidasen und Mischungen davon.

In bevorzugen Enzymformulierungen ist das Enzym ausgewählt unter endo-1,4-β-Xylanasen (EC 3.2.1.8), endo-1,4-β-Glucanasen (EC 3.2.1.4 )und Mischungen davon.

Gegenstand der Erfindung sind auch Enzymformulierungen, welche wenigsten eine weitere der folgenden Eigenschaften aufweisen:
a) Gravimetrischer Staubwert (bestimmt nach einer in den Beispielen beschriebenen Methode) im Bereich von 0 bis 0,5 oder 0,001 bis 0,3 oder 0.001 bis 0.2 oder 0,01 bis 0,2 Gew.-%;
b) Schüttdichte im Bereich von 200 bis 700, 300 bis 500 oder 350 bis 450 g/l (bestimmt nach DIN EN ISO 60)
c) Fließverhalten (bestimmt nach Schulze-Ringscher-Versuch) mit einem ff_{c}-Wert im Bereich von 3 bis 30, 5 bis 15 oder 6 bis 10.

Gegenstand der Erfindung sind insbesondere Enzymformulierungen, wobei die Formulierung eine Mischung umfasst von
a) wenigstens einer Enzymzusammensetzung, deren Enzymkomponente ausgewählt ist unter Xylanasen, Glucanasen und Mischungen davon gemäß obiger Definition, im Gemisch mit Magnesiumsulfat, wobei der Magnesiumsulfatanteil etwa 5 bis 25 oder 15 bis 20 Gew.-% bezogen auf das Gesamtgewicht der trockenen Enzymzusammensetzung beträgt;
b) wenigstens einem Weizengrießkleieträger, wobei das Mischungsverhältnis von Enzymzusammensetzung zu Träger im Bereich von 1 : 5 bis 1 : 500 oder 1:10 bis 1:100 liegt;
c) Pflanzenöl in einem Anteil von etwa 0,1 bis 1, oder 0,3 bis 0,6 Gew.-% bezogen auf das Endgewicht der Enzymformulierung,
   wobei die mittlere Partikelgröße von Enzymzusammensetzung und Träger im Bereich von etwa 100 bis 500 oder 150 bis 350 µm liegt, und der Xylanase-Anteil bei etwa 3.000- 30.000 oder 5.200 bis 18.000 oder 5.400 bis 9.000 TXU/g Formulierung und der Glucanase-Anteil bei etwa 2.000 bis 20.000 oder 2.200 bis 10.000 TGU/g Formulierung liegt. Der prozentuale Anteil der Xylanase liegt bei etwa 1-20 Gew.-%, bevorzugt 2-10 Gew.-% und insbesondere 2,5-5 Gew.-% und an Glucanase bei etwa 0,01-10 Gew.-%, bevorzugt 0,1-6 Gew.-% und insbesondere 0,2-2 Gew.-%.

Besonders bevorzugt sind Formulierungen, enthaltend eine Enzymzusammensetzung des oben beschriebenen Typs, deren Enzymkomponente eine Xylanase ist.

Besonders bevorzugt sind Formulierungen, enthaltend eine Enzymzusammensetzung des oben beschriebenen Typs, deren Enzymkomponente eine Glucanase ist.

Besonders bevorzugt sind Formulierungen, enthaltend eine Enzymzusammensetzung des oben beschriebenen Typs, deren Enzymkomponente eine Mischung aus Xylanase und Glucanase ist.

Besonders bevorzugt sind Formulierungen, umfassend zwei Enzymzusammensetzungen verschiedener Enzyme, wobei die eine Enzymkomponente eine Glucanase und die andere eine Xylanase ist.

Gegenstand der Erfindung sind auch Verfahren zur Herstellung fester Enzymformulierungen nach obiger Definition, wobei man wenigstens eine partikelförmige Enzymzusammensetzung, umfassend wenigstens ein Enzym und wenigstens ein organisches oder anorganisches Salz eines ein- oder zweiwertigen Metall-Kations, mit einem partikelförmigen anorganischen oder organischen physiologisch verträglichen Träger vermischt und das Gemisch mit einer hydrophoben Flüssigkeit (mit einem Schmelzpunkt im Bereich von -60 bis 30 °C gemäß obiger Definition) benetzt.

Insbesondere sind Gegenstand der Erfindung Verfahren, wobei man
a) eine partikelförmige Enzymzusammensetzung, enthaltend ein Enzym, insbesondere Xylanase oder Glucanase, im Gemisch mit wenigstens einem organischen oder anorganischen Salz eines ein- oder zweiwertigen Kations bereitstellt; oder
b) eine partikelförmige Enzymzusammensetzung, enthaltend mindestens zwei voneinander verschiedene Enzyme, ausgewählt unter Xylanase und Glucanase, im Gemisch mit wenigstens einem organischen oder anorganischen Salz eines einoder zweiwertigen Kations bereitstellt; oder
c) mindestens zwei voneinander verschiedene partikelförmige Enzymzusammensetzungen bereitstellt, wobei sich beide Zusammensetzungen dadurch unterscheiden, dass sie mindestens ein unterschiedliches Enzym enthalten, wobei die Enzyme in jeder Zusammensetzung im Gemisch mit wenigstens einem organischen oder anorganischen Salz eines ein- oder zweiwertigen Kations vorliegen.

Bevorzugte Verfahren sind solche, wobei man die Enzymzusammensetzung durch Sprühtrocknung oder durch Sprühtrocknung und Agglomeration einer Enzym-haltigen Flüssigkeit erhält, in welcher wenigstens ein organisches oder anorganisches Salz eines ein- oder zweiwertigen Kations aufgenommen ist.

Bevorzugte Verfahren sind weiterhin solche, wobei man wenigstens zwei Enzymzusammensetzungen voneinander verschiedener Enzyme durch Sprühtrocknung oder durch Sprühtrocknung und Agglomeration wenigstens zweier verschiedener Enzym-haltiger Flüssigkeiten erhält, in welchen wenigstens ein organisches oder anorganisches Salz eines ein- oder zweiwertigen Kations aufgenommen ist, und wobei man
a) jede der wenigstens zwei Enzymzusammensetzungen mit einem partikelförmigen anorganischen oder organischen Träger vermischt, oder
b) einen partikelförmigen anorganischen oder organischen Träger mit den wenigstens zwei Enzymzusammensetzungen vermischt; und
   das gemäß Variante a) oder Variante b) anfallende Gemisch mit einer hydrophoben Flüssigkeit benetzt.

Die verwendete Enzym-haltige Flüssigkeit umfasst dabei wenigstens eine Xylanase, wenigstens eine Glucanase oder ein Gemisch davon.

Insbesondere liegt dabei der Salzanteil in der verwendeten Enzymzusammensetzung im Bereich von 1 bis 30 Gew.-% oder bei etwa 10 bis 25 oder 15 bis 20 Gew.-% bezogen auf das Gesamtgewicht der Enzymzusammensetzung.

Weiterhin verwendet man insbesondere einen Träger und eine Enzymzusammensetzung, deren Verhältnis der mittleren Partikeldurchmesser im Bereich von etwa 1 bis 8, vorzugsweise 1 bis 4 oder 1 bis 2 oder 1 bis 1,5 liegt.

Die mittlere Partikelgröße von verwendeter Enzymzusammensetzung und verwendetem Träger liegt im Bereich von 50 bis 500 µm oder 150 bis 350 µm. Das Mischungsverhältnis von Enzymzusammensetzung und Träger liegt im Bereich von etwa 1:1000 bis 1:5 oder 1:500 bis 1:10 oder 1:100 bis 1:20.

Die mittlere Partikelgröße kann je nach Größe der Partikel entweder mittels einer Siebanalyse (z.B. mit Rüttelsiebmaschine Typ Vibro VS 1000 der Fa. Retsch) ermittelt werden oder auch mittels Laserbeugung (z.B. mittels eines Mastersizers der Fa. Malvern).

Der Anteil der hydrophoben Flüssigkeit beträgt 0,1 bis 5 Gew.-% oder 0,2 bis 2, 0,3 bis 1,5 oder 0,3 bis 0,7 Gew.-%, bezogen auf das Gesamtgewicht der Enzymformulierung.

Gegensand der Erfindung ist insbesondere ein Verfahren zur Herstellung einer festen Enzymformulierung, umfassend wenigstens ein Enzym, ausgewählt unter Xylanasen, Glucanasen und Mischungen davon, wobei man
a) wenigstens eine Enzym-haltige Flüssigkeit zu wenigstens eine Enzymzusammensetzung sprühtrocknet oder sprühtrocknet und agglomeriert, wobei deren Enzymkomponente ausgewählt ist unter Xylanasen, Glucanasen und Mischungen davon, und diese Enzymkomponente im Gemisch mit Magnesiumsulfat in der Flüssigkeit enthalten ist und wobei der Magnesiumsulfatanteil etwa 10 bis 25 Gew.-% bezogen auf das Gesamtgewicht der trockenen Enzymzusammensetzung beträgt;
b) die so erhaltene Enzymzusammensetzung mit einem partikelförmigen anorganischen oder organischen Träger vermischt; und
c) das Enzym/Träger-Gemisch mit einer hydrophoben Flüssigkeit mit einem
   Schmelzpunkt zwischen -60 und 30 °C benetzt.

Bevorzugte Verfahrensvarianten sind dadurch gekennzeichnet, dass man
a) eine partikelförmige Enzymzusammensetzung, enthaltend wenigstens eine Xylanase im Gemisch mit Magnesiumsulfat bereitstellt; oder
b) eine partikelförmige Enzymzusammensetzung, enthaltend wenigstens eine Glucanase im Gemisch mit Magnesiumsulfat bereitstellt; oder
c) eine partikelförmige Enzymzusammensetzung, enthaltend mindestens eine Xylanase und mindestens eine Glucanase im Gemisch mit Magnesiumsulfat bereitstellt; oder
d) mindestens zwei voneinander verschiedene partikelförmige Enzymzusammensetzungen bereitstellt, wobei eine der Zusammensetzungen wenigstens eine Xylanase und die andere der Zusammensetzungen wenigstens eine Glucanase enthält, wobei die Enzyme in jeder Zusammensetzung im Gemisch mit Magnesiumsulfat vorliegen.

In einer besonderen Ausgestaltung des Verfahrens mischt man eine Enzymzusammensetzung mit wenigstens einem Weizengrießkleieträger, wobei das Mischungsverhältnis von Enzymzusammensetzung zu Träger im Bereich von 1 : 5 bis 1 : 500 oder 1:10 bis 1:100 liegt.

Während des Mischens gibt man insbesondere Pflanzenöl in einem Anteil von etwa 0,1 bis 1 Gew.-% oder 0,3 bis 0,6 Gew.-% bezogen auf das Endgewicht der Enzymformulierung zu. Die mittlere Partikelgröße von verwendeter Enzymzusammensetzung und verwendetem Träger liegt dabei insbesondere im Bereich von etwa 100 bis 500 µm oder 150 bis 40 µm und der Xylanase-Anteil beträgt etwa 5.000 - 30.000 oder 5.200 bis 10.000 oder 5.400 bis 9.000 TXU/g Formulierung und/ oder der Glucanase-Anteil etwa 2.000 bis 10.000 oder 2.200 bis 6.000 TGU/g Formulierung.

Gegenstand der Erfindung ist auch die Verwendung einer trockenen Enzymformulierung nach obiger Definition zur Herstellung eines Nahrungsmittels, Nahrungsergänzungsmittels oder eines Tierfutters.

Gegenstand der Erfindung sind auch Verfahren zum Herstellung von Tierfutter, Nahrungsmittel oder Nahrungsergänzungsmittel enthaltend eine trockenen Enzymformulierung nach obiger Definition; insbesondere Tierfutter, umfassend die Herstellung eine
erfindungsgemäßen Enzymformulierung und deren anschließende Einarbeitung in Tierfutter, Nahrungsmittel oder Nahrungsergänzungsmiltel, insbesondere in einem Anteil von etwa 0,001 bis 1 Gew.-%.

### b) Enzyme

Die erfindungsgemäß eingesetzten Enzyme unterliegen keinen Beschränkungen und können sowohl natürlichen oder rekombinanten Ursprungs sein. Es können Enzyme aus Pflanzen, aus Pilzen, aus Bakterien oder Hefen sein. Bevorzugt sind Enzyme aus mikrobiologischen Quellen wie Bakterien, Hefen oder Pilzen. Das Enzym kann aus dem jeweiligen Mikroorganismus nach bekannten Techniken gewonnen werden, die typischerweise die Fermentation des Enzym-produzierenden Mikroorganismus in einem geeigneten Nährmedium und die anschließende Gewinnung des Enzyms oder Enzymkonzentrats aus dem Fermentationsmedium nach Standardtechniken umfasst.

Falls erforderlich können zur pH-Wert-Einstellung der Enzymlösung oder des Enzymkonzentrats übliche Substanzen, wie Puffer, Basen, Säuren, den Formulierungen zugesetzt werden; bevorzugte pH-Werte 3,5 bis 7, besonders bevorzugt 3,5 bis 5 und insbesondere 4 bis 4,5.

Weiterhin können Enzymmutanten oder Enzyme verwendet werden, die eine erhöhte Thermostabilität aufweisen, wie z.B. in den WO's 95/2997, 97/00020, 97/20920, 97/22691, 98/28410 oder 03/062409 vorgeschlagen.

Bevorzugt verwendet man aber erfindungsgemäß als Enzyme Polypeptide mit Xylanase-Aktivität, Polypetide mit Glucanase-Aktivität und Mischungen davon.

### b1) Polypeptide mit Xylanase-Aktivität

Hierbei handelt es sich um Enzyme der Klasse EC 3.2. 1.8 mit der offiziellen Bezeichnung endo-1, 4-beta-Xylanase. Der systematische Name ist 1, 4-beta-D-Xylanxylanohydrolase. Andere Namen sind ebenfalls in Verwendung: endo- (1-4)-beta-Xylanase; (1-4)-beta-xylan 4-Xylanohydrolase ; ende-1; 4-Xylanase ; Xylanase ; beta-1, 4-Xylanase ; endo-1, 4-Xylanase ; endo-beta-1, 4-Xylanase ; endo-1, 4-beta-D-Xylanase ; 1, 4-beta-Xylan Xylanohydrolase ; beta-Xylanase ; beta-1, 4-Xylan Xylanohydrolase ; endo-1,4- beta-Xylanase ; beta-D-Xylanase. Das Enzym katalysiert die Endohydrolyse von 1,4-beta-D- xylosidischen Bindungen in Xylanen.

Die Xylanase kann z.B. von Bakterien abgeleitet sein, wie z.B. von solchen der Gattungen Clostridium, Streptomyces, Paenibacillus, Pseudomonas, Thermoascus, Thermotoga, Bacillus, und z.B. Xylanasen aus folgenden Stämmen Bacillus halodurans, Bacillus pumilus, Bacillus agaradhaerens, Bacillus circulans, Bacillus polymyxa, Bacillus sp., Bacillus stearothermophilus, oder Bacillus subtilis.

Pilz-Xylanasen sind beispielsweise abgeleitet von Hefen und filamentösen Pilzen, wie z.B. aus folgenden Gattungen: Aspergillus, Aureobasidium, Emericella, Fusarium, Gaeumannomyces, Humicola, Lentinula, Magnaporthe, Neocallimastix, Nocardiopsis, Orpinomyces, Paecilomyces, Penicillium, Pichia, Saccharomyces, Schizophyllum, Talaromyces, Thermomyces, Trichoderma, wie z.B. Talaromyces emersonii.

Die Bestimmung der Xylanase-Aktivität erfolgt in an sich bekannter Weise und ist beispielsweise beschrieben in Engelen et al, Journal of AOAC International Vol. 79, No. 5, 1019 (1996). Im Unterschied zu der dort beschriebenen Methode wird anstelle des Xylan-Substrats aus Haferspelzen ( Serva Feinbiochemia GmbH u. Co., Heidelberg) Arabinoxylan aus Weizen (Megazyme, article P-WAXY, Irland) verwendet. Die Herstellung der Substratlösung erfolgt jeweils frisch durch klumpenfreies Auflösen von 1,000 g Arabinoxylan in 100,00 ml Wasser über einen Zeitraum von mindestens 12 Stunden.

### b2) Polypetide mit Glucanase-Aktivität

Endoglucanasen werden als EC 3.2.1.4 klassifiziert und häufig als Cellulasen bezeichnet. Andere Bezeichnungen sind endo-Glucanase, endo-1, 4-beta-Glucanase, Cellulase A oder Carboxymethylcellulase. Die Enzyme katalysieren die Endohydrolyse von 1, 4-beta-D-glucosidischen Bindungen in Cellulose sowie die 1, 4-Verknüpfungen in beta-D-Glucanen, die zudem 1, 3-Verknüpfungen enthalten.

Die Glucanase kann z.B. von Bakterien abgeleitet sein, wie z.B. von solchen der Gattungen Bacillus, Clostridium, Paenibacillus, Pseudomonas, Streptomyces, Thermoascus, Thermotoga. Pilz-Glucanasen sind beispielsweise abgeleitet von Hefen und filamentösen Pilzen, wie z.B. aus folgenden Gattungen: Aspergillus, Aureobasidium, Emericella, Fusarium, Gaeumannomyces, Humicola, Lentinula, Magnaporthe, Neocallimastix, Nocardiopsis, Orpinomyces, Paecilomyces, Penicillium, Pichia, Saccharomyces, Schizophyllum, Talaromyces, Thermomyces, Trichoderma, wie z.B. Talaromyces emersonii.

Die Bestimmung der Glucanase-Aktivität erfolgt in an sich bekannter Weise und ist beispielsweise beschrieben in Engelen et al, Journal of AOAC International Vol. 79, No. 5, 1019 (1996). Im Unterschied zu der dort beschriebenen Methode wird anstelle des Beta-Glukan-Substrats aus Gerste ( Sigma Chemical Co., St. Louis, MO: No. G-6513) Beta-Glukan aus Gerste (Megazyme, article P-BGBM, Irland) verwendet. Die Herstellung der Substratlösung erfolgt jeweils frisch zunächst durch Suspendieren von 0,750 g Glukan in 20 ml Wasser und anschließendem Auflösen durch Zugabe von 20 ml Natriumhydroxidlösung (2 mol/l) unter 15 minütigem Rühren. Es werden 42,5 ml Zitronensäurelösung (1 mol/l) zugesetzt, der pH-Wert mittels Natriumhydroxidlösung (2 mol/l) oder Zitronensäurelösung (1 mol/l) auf 3,50 +/- 0,03 bei 40,0 °C +/- 0,1 °C eingestellt. Nach Abkühlen auf Raumtemperatur wird mit Wasser auf 100,00 ml aufgefüllt.

### c) Stabilisierende Salze

Als Beispiele für geeignete stabilisierende Zusätze sind anorganische oder organische Salze zu nennen.

Insbesondere sind dies Metallsalze, insbesondere Alkali- und Erdalkalimetallsalze organischer Säuren, wie z.B. Mg-, Ca-, Zn-, Na-, K- Salze ein- oder mehrwertiger Carbonsäuren mit 1 bis 8 Kohlenstoffatomen, wie z.B. Citrate, Acetate, Formiate und Hydrogenformiate, außerdem anorganische Salze, wie z.B. Mg-, Ca-, Zn-, Na-, K-sulfate, -carbonate, -silikate oder -phosphate; Erdalkalimetalloxide, wie CaO und MgO; anorganische Pufferungsmittel, wie Alkalimetallhydrogenphosphate, insbesondere Natrium- und Kaliumhydrogenphosphate, wie z.B. K₂HPO₄, KH₂PO₄ und Na₂HPO₄. Besonders bevorzugt verwendet man die folgenden Salze in den angegebenen Gewichtanteilen bezogen auf die Enzymzusammensetzung:
Zinksulfat (0,5 bis 10 oder 3 bis 8 Gew.-%)
Calciumsulfat (1 bis 30 oder 10 bis 25 Gew.-%)
Magnesiumsulfat (5 bis 30 oder 10 bis 25 Gew.-%)
Natriumsulfat (1 bis 30 oder 10 bis 20 Gew.-%)

### d) GeeigneteTräger

Beispiele für Trägermaterialien sind Kohlenhydrate, insbesondere Zucker sowie Stärken, z.B. aus Mais, Reis, Kartoffel, Weizen und Cassave; modifizierte Stärken, z.B. Octenylsuccinatanhydrid; Cellulose und mikrokristalline Cellulose; anorganische Mineralien oder Lehm, z.B. Ton, Kohle, Kieselgur, Kieselsäure, Talc und Kaolin; Grieß, z.B. Weizengrieß, Kleie, z.B. Weizenkleie oder Weizengrießkleie, Mehle; Salze wie Metallsalze, insbesondere Alkali- und Erdalkalimetallsalze organischer Säuren, z.B. Mg-, Ca-,Zn-, Na-, K-Citrat, -acetat, -formiat und -hydrogenformiate, anorganische Salze, z.B. Mg-, Ca-, Zn-, Na-, K-sulfate, -carbonate, -silikate oder -phosphate; Erdalkalimetalloxide wie CaO und MgO; anorganische Pufferungsmittel wie Alkalimetallhydrogenphosphate, insbesondere Natrium- und Kaliumhydrogenphosphate, z.B. K₂HPO₄, KH₂PO₄ und Na₂HPO₄.

### e) Geeignete hydrophobe Flüssigkeiten

Als Beispiele für geeignete hydrophobe Flüssigkeiten sind zu nennen:
Grundsätzlich sind alle hydrophoben Flüssigkeiten (mit einem Schmelzpunkt im Bereich von -60 bis 30 °C, welche einen hydrophoben Molekülteil aufweisen) brauchbar, solange sie als Nahrungs- oder Futtermittelzusatz geeignet sind. Bevorzugt sind natürlich vorkommende pflanzliche oder tierische Flüssigkeiten, wie Phospholipide und Mono-, Di- und Triacylglyceride und Mischungen davon.

Als nichtlimitierende Beispiele sind zu nennen Sojalecithin, pflanzliche Öle, wie z.B. Sonnenblumenöl, Maiskeimöl, Sojaöl, Palmöl, Rapsöl, Palmkernöl, Baumwollsamenöl, Erdnussöl, Babassuöl, Distelöl, sowie tierische Öle, wie z.B. Fischöl.

### f) Herstellung der Formulierung

Die Herstellung der erfindungsgemäßen Enzymformulierungen erfolgt unter Anwendung an sich bekannter Verfahren des Standes der Technik, wie z.B. beschrieben in Mollet et al, Formulierungstechnik, 2000, Verlag Wiley-VCH, Weinheim oder Heinze, Handbuch der Agglomerationstechnik, 2000, Verlag Wiley-VCH, Weinheim.

### f1) Trocknung

Für die Herstellung der salzstabilisierten, vorzugsweise agglomerierten Enzymzusammensetzungen durch Trocknung kommen verschiedene Technologien in Frage, wie insbesondere
- Sprühtrocknung
- Wirbelschichtgranulierung
- Wirbelschichtagglomeration
- FSD-Technologie
- Procell-Technologie von Glatt (WO 2004/108911)

Die Trocknung kann dabei kontinuierlich oder diskontinuierlich (batchweise) erfolgen. Gegebenenfalls muss das getrocknete Produkt nach der Trocknung noch gesiebt, gemahlen oder agglomeriert werden. Kombinationen aus den genannten Schritten sind auch möglich.

Die erfindungsgemäß zur Sprühtrocknung oder Agglomeration eingesetzte Enzymlösung enthält wenigstens ein als Nahrungs- oder Futtermittelzusatz brauchbares Enzym, gelöst oder suspendiert in einer wässrigen Phase, wie z.B. dem Enzymkonzentrat das aus dem Herstellungsprozess bestehend aus Fermentation und Aufarbeitung gewonnen werden kann. Die Lösung besitzt einen Proteinanteil im Bereich von etwa 1 bis 50 Gew.-%, vorzugsweise etwa 10 bis 35 Gew.-%, bezogen auf das Gesamtgewicht der Lösung. Der pH-Wert liegt allgemein im Bereich von etwa 3 bis 9. Neben den oben genannten salzförmigen Enzymstabilisatoren, wie z.B. Alkali- oder Erdalkalimetallsalze, wie Natrium- oder Magnesiumsulfat, kann die Lösung gegebenenfalls weitere übliche Zusätze enthalten. Als Beispiele sind zu nennen: Puffer, wie z.B. Phosphatpuffer; Lösungsvermittler, wie z.B. Ethanol oder oberflächenaktive Mittel und dergleichen.

Für den Fall, dass die Klebeeigenschaften der Enzymlösung nicht ausreichen, um nach dem Aufsprühen ein stabiles Verkleben der Partikel zu gewährleisten, ist zusätzlich die Verwendung eines Bindemittels von Vorteil. Dadurch wird vermieden, dass die Agglomerate beim Trocknen wieder zerfallen. In solchen Fällen ist es bevorzugt, ein in wässrigem Medium lösliches oder dispergierbares Bindemittel in das Wirbelbett einzusprühen. Das Bindemittel kann entweder in der einzusprühenden Enzymlösung gelöst sein oder getrennt davon, gleichzeitig oder zeitlich versetzt, eingesprüht werden. Als Beispiele für geeignete Bindemittel sind zu nennen: Lösungen von Kohlenhydraten, wie z.B. Glucose, Saccharose, Dextrine u.a., Zuckeralkohole, wie z.B. Mannit, oder Polymerlösungen, wie beispielsweise Lösungen von Hydroxypropylmethylcellulose (HPMC), Polyvinylpyrrolidon (PVP), Polyvinylalkohol (PVA), ethoxylierte Cellulose (EC), Ethylcellulose oder Propylcellulose. Durch gezielte Wahl von Menge und Klebeeigenschaften des eingesprühten Bindemittels können Agglomerate unterschiedlicher Größe und Festigkeit hergestellt werden.

Wird das Bindemittel im Gemisch mit dem Enzym aufgesprüht, so liegt der Bindemittelanteil gewöhnlich im Bereich von etwa 0,5 bis 20 Gew.-%, vorzugsweise etwa 1 bis 10 Gew.-%, bezogen auf das Gesamtgewicht der Lösung.

Wird das Bindemittel als separate Lösung aufgesprüht, so liegt der Bindemittelanteil der Lösung im Bereich von etwa 1 bis 30 Gew.-%, bezogen auf das Gesamtgewicht der Lösung. Das Bindemittel liegt hierbei ebenfalls gelöst in einem wässrigen Medium, vorzugsweise keimfreies, entsalztes Wasser, vor. Übliche Zusätze, wie z.B. Puffer, oder Lösungsvermittler können ebenfalls enthalten sein.

Der Anteil des Bindemittels im Endprodukt (d.h. der Enzymzusammensetzung) beträgt erfindungsgemäß 0 bis etwa 20 Gew.-%, beispielsweise etwa 1 bis.6 Gew.-%. Die optimale Menge ist auch von der Art des gewählten Bindemittels abhängig.

Die Sprühtrocknung von flüssigen Enzympräparaten kann in herkömmlicher Weise durchgeführt werden. Dazu wird die Enzymlösung zum Zerstäuber im Sprühturm gepumpt. Die Zerstäubung erfolgt z.B. mittels einer Druckdüse (Einstoffdüse), einer Zweistoffdüse oder eines Zentrifugalzerstäubers. Die Trocknung der Tröpfchen erfolgt durch einen in den Sprühtrockner geleiteten Heißluftstrom. Bei Verwendung von Zentrifugalzerstäubern erfolgt die Trocknung vorzugsweise im Gleichstrom. Bei Düsen kann die Trocknung auch im Gegen- oder Mischstrom erfolgen. Das Pulver kann am Turm ausgetragen werden oder es wird mit dem Luftstrom mitgeführt und in einem Zyklon und/oder Filter abgetrennt. Je nach Produkt und Fahrweise kann eine Nachtrocknung erforderlich sein, die in einem internen, an den Sprühtrockner aufgeflanschten oder einem externen Wirbelbett erfolgen kann.

Das sprühgetrocknete Produkt kann im Anschluss daran in einem Wirbelbett agglomeriert werden. Dazu wird in einem Wirbelbetttrockner pulverförmiges Material, z.B. durch obige Sprühtrocknung erhaltenes Enzympulver, vorgelegt. Die Verwirbelung erfolgt z.B. durch Zufuhr vorgewärmter Luft. Man sprüht auf die Wirbelschicht z.B. eine Enzym-haltige Lösung oder eine Bindemittellösung auf, wodurch man das vorgelegte Pulver mit dieser Lösung benetzt und durch deren Klebeeigenschaften zunehmend agglomeriert. Das Einsprühen in die Wirbelschicht kann von oben (Topspray-Verfahren) oder von unten (Bottomspray-Verfahren) erfolgen. Gleichzeitig wird kontinuierlich oder quasi-kontinuierlich, d.h. intervallweise getaktet, eine Teilmenge Agglomerat aus der Wirbelschicht ausgetragen. Der Austrag wird z.B. mit Hilfe eines Siebs klassiert. Dabei anfallendes Grobgut kann dabei gemahlen und kontinuierlich in das Wirbelbett wieder zurückgeführt werden. Feinanteile, wie z.B. aus der Abluftfilteranlage, können ebenfalls kontinuierlich zurückgeführt werden.

Gemäß einer weiteren Verfahrensvariante kann die Herstellung des erfindungsgemäßen Enzymagglomerats kontinuierlich und zwar unter kontinuierlicher Zuführung einer trockenen pulverförmigen Vorlage, wie z.B. eines trockenen Enzympulvers, in den Wirbelbetttrockner erfolgen. Dafür eignen sich besonders Wirbelbetttrockner mit mehreren Sprüh- und gegebenenfalls Trockenzonen. In der ersten Zone wird trockenes Enzympulver aufgegeben, verwirbelt und Enzymlösung und/oder Bindemittel eingesprüht. Das in dieser Zone gebildete Agglomerat wird in die nächste Zone überführt. In diese und gegebenenfalls in einer oder mehreren weiteren Zonen kann ebenfalls Enzymund/oder Bindemittellösung gleicher oder unterschiedlicher Zusammensetzung eingesprüht werden. Durch einen für alle Zonen gemeinsamen Zuluftstrom oder getrennte Zuluftströme, die entsprechend erwärmt sind, wird das Wasser der aufgesprühten Enzym- oder Bindemittellösung entzogen. In einer oder mehreren der letzten Zonen kann noch nachgetrocknet werden. Hier befindet sich auch der Produktaustrag. Die Aufarbeitung des Produkts erfolgt wie oben beschrieben.

Eine weitere bevorzugte Verfahrensvariante umfasst eine Sprühtrocknung von Enzymlösung, gekoppelt mit der anschließenden Agglomeration des sprühgetrockneten Enzympulvers. Diese kann diskontinuierlich oder kontinuierlich durchgeführt werden. Bevorzugt ist die kontinuierliche Fahrweise.

Derartige Verfahren können unter Verwendung herkömmlicher Sprühtrocknungsanlagen durchgeführt werden. Vorteilhafterweise erfolgt die Durchführung aber in Vorrichtungen, welche als FSD (Fluidized Spray Dryer), SBD (Spray Bed Dryer) oder MSD (Multi Stage Dryer) bekannt sind.

Der anfallende Feinanteil des Pulvers kann hierbei bereits im Sprühtrockner in den Prozess wieder eingebunden werden, wenn man diesen, z.B. nach Abscheidung in einem Zyklon oder Filter, wieder in die feuchte Zone des Trockners zurückführt. Die eigentliche Agglomeration findet dann in einer weiteren Stufe in einem Wirbelbett statt. Diese Stufe kann in den Sprühtrockner integriert sein (internes Wirbelbett) oder sie kann in einer separaten Apparatur (zusätzliches Wirbelbett) durchgeführt werden. In das Wirbelbett kann, falls erforderlich, gleichzeitigem Trocknen weitere Enzymlösung, eine Enzymlösung welche außerdem Bindemittel enthält oder nur Bindemittel in gelöster oder dispergierter Form eingedüst werden, um die Agglomeration zu unterstützen. Beispiele für geeignete Bindemittel für die Agglomeration sind Hydroxypropylmethylcellulose, Polyvinylpyrrolidon, Polyethylenglykole und Blockpolymere von Polyoxyethylen und Polyoxypropylen. Vorzugsweise werden die Verfahrensparameter aber so eingestellt, dass keine weitere Zudosierung zur Agglomerat-Herstellung erforderlich ist. Die Zusammensetzung und Menge der eingedüsten Flüssigkeiten richten sich nach den Klebeeigenschaften der eingesprühten Lösung, der zu erzielenden Agglomeratgrösse und den Prozessbedingungen. Je nach aufgesprühter Menge kann eine Nachtrocknung in einer weiteren Stufe erforderlich sein. Die Aufarbeitung des Produkts erfolgt dann in der oben beschriebenen Weise.

Im Falle einer hohen Temperaturlabilität der sprühgetrockneten Enzyme ist während der erfindungsgemäßen Verfahren die Regelung der Produkttemperatur von besonderer Bedeutung. Sie sollte möglichst niedrig gewählt werden, da mit zunehmender Temperatur und/oder Dauer des Sprühtrocknungs- und Agglomerationsverfahrens die Aktivitätsverluste zunehmen. Typischerweise liegt die Produkttemperatur bei Sprühtrocknung, d.h. die Temperatur des festen sprühgetrockneten Pulvers, bei etwa 40 bis 75 °C, insbesondere bei weniger als etwa 70 °C, häufig weniger als 60 °C. Je länger die Verweilzeit im Wirbelbett ist, um so niedriger sollte die Temperatur gewählt werden.

Die Produkttemperatur während der Agglomeration und Trocknung in der Wirbelschicht, d. h. die Temperatur des im Wirbelbett befindlichen Agglomerates, ist wegen der längeren Verweilzeit in der Vorrichtung niedrig zu wählen und liegt bei Werten von etwa 30 als 70 °C, insbesondere weniger als 60 °C und vorzugsweise bei weniger als 50 °C.

Um den Restfeuchtegehalt weiter zu verringern kann die Durchführung eines Nachtrocknungsschrittes erforderlich sein. Auch während der Nachtrocknung sollte die Produkttemperatur in dem oben genannten Bereich und insbesondere bei 50°C oder weniger liegen. Durch die Nachtrocknung wird der Restfeuchtegehalt in den erfindungsgemäßen Präparaten auf Werte von weniger als etwa 20 Gew.-%, vorzugsweise etwa 5 bis 17 Gew.-% reduziert.

Die Trocknung während der Agglomeration bzw..die Nachtrocknung wird durch Verwendung von vorgewärmter Zuluft erreicht. Die Zulufttemperatur, die je nach gewählter Sollprodukttemperatur, Luftmenge und Sprührate unterschiedlich sein kann, liegt im Allgemeinen in einem Bereich zwischen 30 und 180 °C. Die Nachtrocknung erfolgt bei niedrigerer Temperatur, nämlich im Bereich von etwa 35 bis 55 °C.

Die Dauer der Agglomeration ist ebenfalls von der Größe des gewählten Ansatzes abhängig liegt aber etwa im Bereich von 30 Minuten bis mehreren Stunden.

### f2) Herstellung der Enzymformulierung

Unter Anwendung an sich bekannter Mischtechniken wird das sprühgetrocknete, ggf. agglomerierte Vorprodukt (trockene Enzymzusammensetzung) mit dem oben beschriebenen Trägermaterial vermischt. Dazu gibt man , z.B. portionsweise, das Enzympräparat zu Träger hinzu und mischt, falls erforderlich einige Zeit, z.B. 1 bis 5 Minuten, bis eine gleichmäßige Verteilung erreicht ist. Dann wird die hydrophobe Flüssigkeit zugesetzt. Dies kann während des Mischvorgangs auf oder in das Gemisch gesprüht, getropft oder gegossen werden. Nach beendeter Zugabe wird der Mischvorgang fortgesetzt, beispielsweise 5 bis 45 Minuten, bis das Öl gleichmäßig verteilt ist. Das dabei anfallende Produkt besitzt einen sehr geringen Staubanteil. Weitere Behandlungsschritte sind gewöhnlich nicht erforderlich.

Verschiedene Mischertypen sind für die Abmischung geeignet, wie z.B. Konus - Schneckenmischer (z.B. Fa. Nauter), Pflugscharmischer (z.B. Fa. Lödige), Zweiwellenmischer. Die Mischzeiten hängen von dem gewählten Mischertyp ab und können sich unterscheiden.

### g) Nahrungs- und Futtermittelzusanimensetzungen

Die erfindungsgemäß hergestellten Enzymformulierungen eignen sich insbesondere zur Additivierung von Nahrungs- und Futtermitteln.

Besonders geeignet sind die Formulierungen als Zusätze zu Tierfutter, im Gemisch mit Einzelfuttermitteln pflanzlichen oder tierischen Ursprungs gemäß FMV (Futtermittelverordnung), wie z.B. Getreidefolgeprodukte , Weizenfuttermehl, Weizenkleie; Extraktionsschrote, Trester, Melasseschnitzel, Fischmehl, Fleischknochenmehle; und/oder mineralischen Einzelfuttermitteln gemäß FMV , wie z.B. Carbonate, Phosphate, Sulfate, Propionate. Ebenfalls geeignet sind Getreide, wie Weizen , Roggen, Gerste, Hafer, Mais, Hirse oder Triticale; Getreidefolgeprodukte (Nebenprodukte der Müllerei), wie Kleien, Grießkleien, Weizengrießkleien, Futtermehle oder Nachmehle; Nebenprodukte aus der Ölgewinnung (Extraktionsschrote, Expeller, Kuchen); Nebenprodukte aus der Gewinnung von Zucker (Melasse, Trockenschnitzel, Futterzucker, Pülpe, Katoffelstärke, Maiskleber, Weizenkleber); Nebenerzeugnisse des Gärungsgewerbes, Biertreber, Hefe, Malzkeime, Schlempe; sowie tierische und sonstige Futtermittel, wie Blutmehl, Fischmehl, Presssaft, Kartoffeleiweiß.

### Experimenteller Teil

### Herstellungsbeispiel V1: Xylanase-Formulierung

a) In einem wässrigen Xylanasekonzentrat mit einem Trockenmassegehalt von etwa 20 bis 35 Gew.-%, einem pH-Wert im Bereich von 3,5-5,0 und einer Aktivität von 60 000 bis 100 000 TXU/g löste man bei 4-10 °C 10 - 20 Gew.-% Magnesiumsulfat-Heptahydrat, bezogen auf das Konzentrat.
b) Für die Sprühtrocknung und Agglomeration wurde die unter a) hergestellte Enzymzusammensetzung in einem Laborwirbelbett Aeromat Typ MP-1 der Firma Niro-Aeromatic über eine 2-Stoffdüse nach dem Top-Spray-Verfahren gesprüht. Der Kunststoffkonus des Wirbelbetts hat einen Anströmboden-Durchmesser von 110 mm und einen Lochboden mit 12 % freier Fläche. Das Wirbelbett wurde mit einer Luftmenge von 50 m³/h und Zulufttemperaturen von 40 bis 100°C beaufschlagt. Die Zulufttemperatur wurde geregelt, so dass das Produkt in der Wirbelschicht eine Temperatur von ca. 45°C hielt. Die Sprühdauer betrug 240 Min. Anschließend wurde das Produkt unter Wirbeln bei 50 m³/h Zuluft auf 30 °C abgekühlt.
c) Die unter b) gewonnene Enzymzusammensetzung wurde ausgesiebt. Feingut und Grobgut wurden abgesiebt, so dass man eine Nutzfraktion mit einer Partikelgrößenverteilung von 100 µm bis 400 µm erhielt.
Man erhielt ein Produkt mit folgenden Kenndaten:
Zusammensetzung:

| | |
|---|---|
| Xylanase (Trockenmasse) | 65 Gew.-% |
| Magnesiumsulfat (MgSO₄) | 20 Gew.-% |
| Restfeuchte | 15 Gew.-% |
| Aktivität | von 200 000 bis 300 000 TXU/g |
| Aussehen (Mikroskop) | Agglomerate bestehend aus mehreren Primärpartikelchen |
| Mittlerer Partikeldurchmesser | 171 µm |

d) Zur Herstellung der Enzymformulierung wurde Weizengrießkleie (675,5 g) in einem Labormischer (Fa. Lödige) vorgelegt und bei Raumtemperatur und 170 Umdrehungen pro Minute homogenisiert. Bei diesen Bedingungen wurden 21 g der unter c) hergestellten Enzymzusammensetzung in den Mischer gegeben und 5 min gemischt.

Danach wurden 3,5 g Sojaöl langsam über eine Pipette zugetropft und danach 30 min. nachgemischt.
Man erhielt ein Produkt mit folgenden Kenndaten:
Zusammensetzung:

| | |
|---|---|
| Weizengrießkleie (Trockenmasse) | 90 Gew.-% |
| Enzymzusammensetzung (aus c)) | 3 Gew.-% |
| Sojaöl | 0,5 Gew.-% |
| Restfeuchte | 6,5 Gew.-% |
| Aktivität | von 5 000 bis 7 000 TXU/g |
| Mittlerer Partikeldurchmesser: | 337 µm |

### Herstellungsbeispiel V2: Glucanase-Formulierung

a) In einem wässrigen β-Glucanasekonzentrat mit einem Trockenmassegehalt von etwa 20 bis 35 Gew.-%, einem pH-Wert im Bereich von 3,5-5,0 und einer Aktivität von 150 000 bis 400 000 TGU/g löste man bei 4-10 °C 10 bis 20 Gew.-% Magnesiumsulfat-Heptahydrat, bezogen auf das Konzentrat.
b) Für die Sprühtrocknung und Agglomeration wurde die unter a) hergestellte Enzymzusammensetzung in einem Laborwirbelbett Aeromat Typ MP-1 der Firma Niro-Aeromatic über eine 2-Stoffdüse nach dem Top-Spray-Verfahren gesprüht. Der Kunststoffkonus des Wirbelbetts hat einen Anströmboden-Durchmesser von 110 mm und einen Lochboden mit 12 % freier Fläche. Das Wirbelbett wurde mit einer Luftmenge von 50 m³/h und Zulufttemperaturen von 40 bis 100 °C beaufschlagt. Die Zulufttemperatur wurde geregelt, so dass das Produkt in der Wirbelschicht eine Temperatur von ca. 45 °C hielt. Die Sprühdauer betrug 240 min. Anschließend wurde das Produkt unter Wirbeln bei 50 m³/h Zuluft auf 30 °C abgekühlt.
c) Die unter b) gewonnene Enzymzusammensetzung wurde ausgesiebt. Feingut und Grobgut wurden abgesiebt, so dass man eine Nutzfraktion mit einer Partikelgrößenverteilung von 100 µm bis 400 µm erhielt.
Man erhielt ein Produkt mit folgenden Kenndaten:
Zusammensetzung:

| | |
|---|---|
| Glucanase (Trockenmasse) | 65 Gew.-% |
| Magnesiumsulfat (MgSO₄) | 20 Gew.-% |
| Restfeuchte | 15 Gew.-% |
| Aktivität | von 500 000 bis 120 000 TGU/g |
| Aussehen (Mikroskop) | Agglomerat bestehend aus mehreren Primärpartikelchen |
| Mittlerer Partikeldurchmesser | 167 µm |

d) Zur Herstellung der Enzymformulierung wurde Weizengrießkleie (693 g) in einem Labormischer (Firma Lödige) vorgelegt und bei Raumtemperatur und 170 Umdrehungen pro Minute homogenisiert. Bei diesen Bedingungen wurden 3,5 g der unter c) hergestellten Enzymzusammensetzung in den Mischer gegeben und 5 min gemischt. Danach wurden 3,5 g Sojaöl langsam über eine Pipette zugetropft und danach 30 min nachgemischt.
Man erhielt ein Produkt mit folgenden Kenndaten:
Zusammensetzung:

| | |
|---|---|
| Weizengrießkleie (Trockenmasse) | 92,5 Gew.-% |
| Enzymzusammensetzung (aus c)) | 0,5 Gew.-% |
| Sojaöl | 0,5 Gew.-% |
| Restfeuchte | 6,5 Gew.-% |
| Aktivität | von 1 000 bis 7 000 TGU/g |
| Mittlerer Partikeldurchmesser | 321 µm |

### Herstellungsbeispiel V3: Xylanase/Glucanase-Formulierung

Zur Herstellung einer Enzymformulierung wurde Weizengrießkleie (672 g) in einem Labormischer (Firma Lödige) vorgelegt und bei Raumtemperatur und 170 Umdrehungen pro Minute homogenisiert. Bei diesen Bedingungen wurden 21 g der unter Herstellungsbeispiel V1 c) hergestellten Enzymzusammensetzung und 3,5 g der unter Herstellungsbeispiel V2 c) hergestellten Enzymzusammensetzung in den Mischer gegeben und 5 min gemischt. Danach wurden 3,5 g Sojaöl langsam über eine Pipette zugetropft und anschließend 30 min. nachgemischt.
Man erhielt ein Produkt mit folgenden Kenndaten:
Zusammensetzung:

| | |
|---|---|
| Weizengrießkleie (Trockenmasse) | 89,5 Gew.-% |
| Enzymzusammensetzung (aus V1 c)) | 3 Gew.-% |
| Enzymzusammensetzung (aus V2 c)) | 0,5 Gew.-% |
| Sojaöl | 0,5 Gew.-% |
| Restfeuchte | 6,5 Gew.-% |
| Xylanaseaktivität | von 5 000 bis 7 000 TXU/g |
| Glucanase-Aktivität | von 1 000 bis 7 000 TGU/g |
| Mittlerer Partikeldurchmesser | 328 µm |

### Herstellungsbeispiel V4: Xylanase/Glucanase-Formulierung

a) Ein wässriges β-Glucanasekonzentrat mit einem Trockenmassegehalt von etwa 20 bis 35 Gew.-%, einem pH-Wert im Bereich von 3,5-5,0 und einer Aktivität von 150 000 bis 400 000 TGU/g wurde mit einem wässrigen Xylanasekonzentrat mit einem Trockenmassegehalt von etwa 20 bis 35 Gew.-%, einem pH-Wert im Bereich von 3,5 bis 5,0 und einer Aktivität von 60 000 bis 100 000 TXU/g im Verhältnis 1:8 gemischt. In der Mischung löste man bei 4-10 °C 10 bis 30 Gew.-% Magnesiumsulfat-Heptahydrat, bezogen auf das Konzentrat.

Nachfolgend wurde das unter a) gewonnene Enzymkonzentrat wie im Herstellungsbeispiel V1 in den Schritten b) bis d) weiterverarbeitet.

Man erhielt ein Produkt mit folgenden Kenndaten:
Zusammensetzung:

| | |
|---|---|
| Weizengrießkleie (Trockenmasse) | 90 Gew.-% |
| Enzymzusammensetzung (aus c)) | 3 Gew.-% |
| Sojaöl | 0,5 Gew.-% |
| Restfeuchte | 6,5 Gew.-% |
| Xylanaseaktivität | von 5 000 bis 7 000 TXU/g |
| Glucanase-Aktivität | von 1 000 bis 7 000 TGU/g |
| Mittlerer Partikeldurchmesser | 343 µm |

### Testbeispiel 1: Bestimmung des Staubwerts

Der Staubwert (% bezogen auf die Gesamtmenge des Produkts) von erfindungsgemäßen Abmischungen wird mit und ohne Ölzusatz bestimmt.

Die Bestimmung erfolgt nach folgender Methode:
Je drei Proben zu je 10 ± 0,03 g des zu untersuchenden Feststoffs werden über ein Fallrohr (Länge = 60 cm; Durchmesser = 3 cm) langsam (c.a. 2 bis 3 Sekunden) in ein Behältnis (20,2 cm Höhe, 19,5 cm Breite,19,5 cm Länge; ein Absaugschlauch befindet sich an einer Seitenwand in einer Höhe von ca.13 cm und ist im rechten Winkel (90°) zu dem Fallrohr angebracht) geschüttet. Mit Hilfe einer über den Ansaugschlauch mit dem Behältnis verbundenen Ölpumpe wird mit konstanter Geschwindigkeit (15 ± 0,5 l/min) für 1 Minute der dadurch entstandene Staub aus dem Behältnis abgesaugt und auf einem Filter aufgefangen. Dazu verwendet man eine Glasnutsche (Durchmesser 35 mm, D2, 50ml) versehen mit einem geeigneten Filter (z.B. Sartorius Glasfaser-Prefilter, 13 400-37-S; Durchmesser 35 mm). Die abgesaugte Stabmenge wird mit Hilfe einer Analysenwaage bestimmt, in Relation zur eingesetzten Probenmenge gebracht und als prozentualer Mittelwert ausgedrückt. Je nach ermitteltem prozentualen Staubwerte wird das Staubverhalten der Proben wie folgt beschrieben:

| Staubwert [%] | Beschreibung |
|---|---|
| 0 - 0,05 | nahezu staubfrei |
| 0,05 - 0,25 | schwach staubend |
| 0,25 - 1,00 | staubend |
| > 1,00 | stark staubend |

### Einsatzstoffe:

Xylanasepulver (XEA) : Aktivität: 229300 TXU/g; Mittlerer Partikeldurchmesser = 171; (20 Gew.-% Magnesiumsulfat-Heptahydrat); getrocknet analog zu Herstellungsbeispiel V1

Weizengrießkleie (WGK) (Hildebrandmühlen); Mittlerer Partikeldurchmesser = 370 Sojaöl

### Abmischung der Proben:

Die WGK wird im Lödigemischer vorgelegt, das SD-Pulver dazugegeben und bei Raumtemperatur und 5 min bei 170 U/min vorgemischt. Das Sojaöl wird auf ca. 80°C erwärmt, mit einer feinen Pipette langsam zugetropft und 30min. nachgemischt. Es werden jeweils 1000 g Abmischung hergestellt. Die ermittelten Staubwerte für verschiedene Abmischungen sowie für reines XEA und reine WGK sind in folgender Tabelle zusammengefasst:

| Probe E5/051 | Weizengrießkleie (g) | SD-Pulver (g) | Sojaöl (g) | Sollaktivität (TXU/g) | Staubwert (%) | Bemerkung | Sojaöl (%) |
|---|---|---|---|---|---|---|---|
| Batch 1 | 967,3 | 32,7 | **0,0** | 7500 | 0,081 | schwach staubend | **0,0** |
| Batch 2 | 962,3 | 32,7 | **5,0** | 7500 | 0,025 | nahezu staubfrei | **0,5** |
| Batch 3 | 957,3 | 32,7 | **10,0** | 7500 | 0,015 | nahezu staubfrei | **1,0** |
| WGK | **pur** | - | **0,0** | 0 | 0,120 | schwach staubend | **0,0** |
| XEA | - | **pur** | **0,0** | 229300 | 0,049 | nahezu staubfrei | **0,0** |

Man beobachtet eine überraschend signifikante Verringerung der Staubneigung von erfindungsgemäßen ölhaltigen Abmischungen.

Weiterhin sind nach visueller Überprüfung sowie lichtmikroskopischer Untersuchung bei den untersuchten Batches keine Unterschiede im Entmischungsverhalten feststellbar (Ergebnisse nicht gezeigt).

### Testbeispiel 2: Bestimmung der Fließfähigkeit

Die Bestimmung des Fließverhaltens erfindungsgemäßer Enzymformulierungen erfolgt nach bekannten Verfahren. Im Stand der Technik sind verschiedene prinzipiell geeignete Methoden beschrieben (vgl. Schmitt et al., Part. Part. Syst. Charact. 21 (2004) 403-410).

Erfindungsgemäß erfolgt die Bestimmung mit Hilfe des Schulze-Ringschertesters RST.01-pc. Der Versuch erfolgte nach der Methode ASTM D6773 (Schulze Ring Shear Tester 2002).

Folgende Testparameter wurden verwendet:
Lagerzeit der Probe in der Messzelle: 0 h
Temperatur: 22 °C
Relative Luftfeuchte: 70 %
Verfestigungsspannung (Auflast): σ₁= 11,18 kPa

Es konnte nach der ASTM D6773 Methode eine Fließfähigkeit von ff_{c}= 8,8 erreicht werden. Damit ist das Produkt gut fließfähig.

## Patentansprüche

1. Feste Enzymformulierung zur Einarbeitung in Tierfutter, Nahrungs- oder Nahrungsergänzungsmittel, umfassend eine Mischung von
a) wenigstens einer partikelförmigen Enzymzusammensetzung von wenigstens einem Enzym und wenigstens einem organischen oder anorganischen Salz eines ein- oder zweiwertigen Kations mit
b) wenigstens einem partikelförmigen anorganischen oder organischen Träger und
c) wenigstens einer hydrophoben Flüssigkeit;
wobei das Verhältnis der mittleren Partikeldurchmesser von Träger zu Enzymzusammensetzung im Bereich von 1 bis 8 liegt und wobei das Mischungsverhältnis von Enzymzusammensetzung und Träger im Bereich von 1:1000 bis 1:5 Gewichtsteilen liegt;
wobei die mittleren Partikelgrößen von Enzymzusammensetzung und Träger unabhängig voneinander jeweils im Bereich von 50 bis 500 µm liegen; und
wobei der Anteil der hydrophoben Flüssigkeit 0,1 bis 5 Gew.-%, bezogen auf das Gesamtgewicht der Enzymformulierung, beträgt.

2. Enzymformulierung nach Anspruch 1, umfassend
eine partikelförmige Enzymzusammensetzung, enthaltend ein Enzym im Gemisch mit wenigstens einem organischen oder anorganischen Salz eines ein- oder zweiwertigen Kations.

3. Enzymformulierung nach Anspruch 1, umfassend eine partikelförmige Enzymzusammensetzung, enthaltend mindestens zwei voneinander verschiedene Enzyme im Gemisch mit wenigstens einem organischen oder anorganischen Salz eines ein- oder zweiwertigen Kations.

4. Enzymformulierung nach Anspruch 1, umfassend mindestens zwei voneinander verschiedene partikelförmige Enzymzusammensetzungen, wobei sich beide Zusammensetzungen dadurch unterscheiden, dass sie mindestens ein unterschiedliches Enzym enthalten, wobei die Enzyme in jeder Zusammensetzung im Gemisch mit wenigstens einem organischen oder anorganischen Salz eines ein- oder zweiwertigen Kations vorliegen.

5. Enzymformulierung nach einem der Ansprüche 2 bis 4, wobei die mittleren Partikelgrößen von Enzymzusammensetzung und Träger unabhängig voneinander jeweils im Bereich von 150 bis 350 µm liegen.

6. Enzymformulierung nach einem der vorhergehenden Ansprüche, wobei der Anteil der hydrophoben Flüssigkeit 0,1 bis 1,5 Gew.-%, bezogen auf das Gesamtgewicht der Enzymformulierung, beträgt.

7. Enzymformulierung nach einem der vorhergehenden Ansprüche, wobei der Salzanteil in der Enzymzusammensetzung im Bereich von 1 bis 30 Gew.-%, bezogen auf das Gesamtgewicht der Enzymzusammensetzung, liegt.

8. Enzymformulierung nach einem der vorhergehenden Ansprüche, wobei das (die) Enzym(e) ausgewählt ist (sind) unter Xylanasen, Glucanasen, Cellulasen, Proteasen, Keratinasen, Amylasen und Mischungen davon.

9. Enzymformulierung nach einem der vorhergehenden Ansprüche, wobei die Formulierung eine Mischung umfasst von
a) wenigstens einer Enzymzusammensetzung, deren Enzymkomponente ausgewählt ist unter Xylanasen, Glucanasen und Mischungen davon, im Gemisch mit Magnesiumsulfat, wobei der Magnesiumsulfatanteil etwa 5 bis 25 Gew.-%, bezogen auf das Gesamtgewicht der trockenen Enzymzusammensetzung, beträgt;
b) wenigstens einem Weizengrießkleieträger, wobei das Mischungsverhältnis von Enzymzusammensetzung zu Träger im Bereich von 1 : 5 bis 1 : 500 liegt; und
c) Pflanzenöl in einem Anteil von 0,1 bis 1 Gew.-%, bezogen auf das Endgewicht der Enzymformulierung,
wobei die mittlere Partikelgröße von Enzymzusammensetzung und Träger im Bereich von etwa 150 bis 500 µm liegt, und der Xylanase-Anteil bei 3.000 - 30.000 TXU/g Formulierung und der Glucanase-Anteil bei 2.000 bis 20.000 TGU/g Formulierung liegt.

10. Enzymformulierung nach Anspruch 9, umfassend eine Enzymzusammensetzung, deren Enzymkomponente eine Xylanase, eine Glucanase, oder eine Mischung aus Xylanase und Glucanase; oder umfassend zwei Enzymzusammensetzungen verschiedener Enzyme, wobei die eine Enzymkomponente eine Glucanase und die andere eine Xylanase ist.

11. Verfahren zur Herstellung einer festen Enzymformulierung nach einem der vorhergehenden Ansprüche, wobei man wenigstens eine partikelförmige Enzymzusammensetzung, umfassend wenigstens ein Enzym und wenigstens ein organisches oder anorganisches Salz eines ein- oder zweiwertigen Kations, mit wenigstens einem partikelförmigen anorganischen oder organischen Träger vermischt und das Gemisch mit einer hydrophoben Flüssigkeit benetzt.

12. Verfahren nach Anspruch 11, wobei man eine partikelförmige Enzymzusammensetzung, enthaltend ein Enzym im Gemisch mit wenigstens einem organischen oder anorganischen Salz eines ein- oder zweiwertigen Kations bereitstellt.

13. Verfahren nach Anspruch 11, wobei man eine partikelförmige Enzymzusammensetzung, enthaltend mindestens zwei voneinander verschiedene Enzyme im Gemisch mit wenigstens einem organischen oder anorganischen Salz eines ein- oder zweiwertigen Kations bereitstellt.

14. Verfahren nach Anspruch 11, wobei man mindestens zwei voneinander verschiedene partikelförmige Enzymzusammensetzungen bereitstellt, wobei sich beide Zusammensetzungen dadurch unterscheiden, dass sie mindestens ein unterschiedliches Enzym enthalten, wobei die Enzyme in jeder Zusammensetzung im Gemisch mit wenigstens einem organischen oder anorganischen Salz eines ein- oder zweiwertigen Kations vorliegen.

15. Verfahren nach einem der Ansprüche 11, 12 oder 13, wobei man die Enzymzusammensetzung durch Sprühtrocknung oder durch Sprühtrocknung und Agglomeration einer Enzym-haltigen Flüssigkeit erhält, in welcher wenigstens ein organisches oder anorganisches Salz eines ein- oder zweiwertigen Kations aufgenommen ist.

16. Verfahren nach Anspruch 11 oder 14, wobei man wenigstens zwei Enzymzusammensetzungen voneinander verschiedener Enzyme durch Sprühtrocknung oder durch Sprühtrocknung und Agglomeration wenigstens zweier verschiedener Enzym-haltiger Flüssigkeiten erhält, in welchen wenigstens ein organisches oder anorganisches Salz eines ein- oder zweiwertigen Kations aufgenommen ist, und wobei man
a) jede der wenigstens zwei Enzymzusammensetzungen mit einem partikelförmigen anorganischen oder organischen Träger vermischt, oder
b) einen partikelförmigen anorganischen oder organischen Träger mit den wenigstens zwei Enzymzusammensetzungen vermischt; und
das gemäß Variante a) oder Variante b) anfallende Gemisch mit einer hydrophoben Flüssigkeit benetzt.

17. Verfahren zur Herstellung einer festen Enzymformulierung, umfassend wenigstens ein Enzym, ausgewählt unter Xylanasen, Glucanasen und Mischungen davon, wobei man
a) wenigstens eine Enzym-haltige Flüssigkeit zu wenigstens einer Enzymzusammensetzung sprühtrocknet oder sprühtrocknet und agglomeriert, wobei deren Enzymkomponente ausgewählt ist unter Xylanasen, Glucanasen und Mischungen davon, und diese Enzymkomponente im Gemisch mit Magnesiumsulfat in der Flüssigkeit enthalten ist und wobei der Magnesiumsulfatanteil 5 bis 25 Gew.-% bezogen auf das Gesamtgewicht der trockenen Enzymzusammensetzung beträgt;
b) die so erhaltene Enzymzusammensetzung mit einem partikelförmigen anorganischen oder organischen Träger vermischt; und
c) das Enzym/Träger-Gemisch mit einer hydrophoben Flüssigkeit benetzt.

18. Verfahren nach Anspruch 17, wobei man
a) eine partikelförmige Enzymzusammensetzung, enthaltend wenigstens eine Xylanase im Gemisch mit Magnesiumsulfat bereitstellt; oder
b) eine partikelförmige Enzymzusammensetzung, enthaltend wenigstens eine Glucanase im Gemisch mit Magnesiumsulfat bereitstellt; oder
c) eine partikelförmige Enzymzusammensetzung, enthaltend mindestens eine Xylanase und mindestens eine Glucanase im Gemisch mit Magnesiumsulfat bereitstellt; oder
d) mindestens zwei voneinander verschiedene partikelförmige Enzymzusammensetzungen bereitstellt, wobei eine der Zusammensetzungen wenigstens eine Xylanase und die andere der Zusammensetzungen wenigstens eine Glucanase enthält, wobei die Enzyme in jeder Zusammensetzung im Gemisch mit Magnesiumsulfat vorliegen.

19. Verfahren nach Anspruch 17 oder 18, wobei man eine oder zwei verschiedene Enzymzusammensetzungen mit wenigstens einem Weizengrießkleieträger mischt, wobei das Mischungsverhältnis von Enzymzusammensetzung zu Träger im Bereich von 1 : 5 bis 1 : 1000 liegt.

20. Verwendung einer trockenen Enzymformulierung nach einem der Ansprüche 1 bis 10, zur Herstellung eines Nahrungsmittels, Nahrungsergänzungsmittels oder eines Tierfutters.

21. Verfahren zur Herstellung von Tierfutter, Nahrungsmittel oder Nahrungsergänzungsmittel enthaltend eine trockene Enzymformulierung nach einem der Ansprüche 1 bis 10, umfassend die Herstellung einer solchen trockenen Enzymformulierung mit Hilfe eines Verfahrens nach einem der Ansprüche 11 bis 19, und deren anschließende Einarbeitung in Tierfutter, Nahrungsmittel oder Nahrungsergänzungsmittel.

## Claims

1. A solid enzyme formulation for incorporating in animal feed, food or food supplement, comprising a mixture of
a) at least one particulate enzyme composition of at least one enzyme and at least one organic or inorganic salt of a monovalent or divalent cation with
b) at least one particulate inorganic or organic support and
c) at least one hydrophobic liquid;
the ratio of the median particle diameter of support to enzyme composition being in the range from 1 to 8 and the mixing ratio of enzyme composition and support being in the range from 1:1000 to 1:5 parts by weight;
the median particle sizes of enzyme composition and support independently of one another each being in the range from 50 to 500 µm; and
the fraction of the hydrophobic liquid being 0.1 to 5 % by weight; based on the total weight of the enzyme formulation.

2. The enzyme formulation according to claim 1, comprising
a particulate enzyme composition comprising an enzyme in a mixture with at least one organic or inorganic salt of a monovalent or divalent cation.

3. The enzyme formulation according to claim 1, comprising a particulate enzyme composition comprising at least two enzymes which are different from one another in a mixture with at least one organic or inorganic salt of a monovalent or divalent cation.

4. The enzyme formulation according to claim 1, comprising least two particulate enzyme compositions which are different from one another, the two compositions differing in that they comprise at least one different enzyme, the enzymes in each composition being present in a mixture with at least one organic or inorganic salt of a monovalent or divalent cation.

5. The enzyme formulation according to one of claims 2 to 4, the median particle sizes of enzyme composition and support independently of one another each being in the range from 150 to 350 µm.

6. The enzyme formulation according to one of the preceding claims, the fraction of the hydrophobic liquid being 0.1 to 15% by weight, based on the total weight of the enzyme formulation.

7. The enzyme formulation according to one of the preceding claims, the salt fraction in the enzyme composition being in the range from 1 to 30% by weight, based on the total weight of the enzyme composition.

8. The enzyme formulation according to one of the preceding claims, the enzyme(s) being selected from xylanases, glucanases, cellulases, proteases, keratinases, amylases and mixtures thereof.

9. The enzyme formulation according to one of the preceding claims, the formulation comprising a mixture of
a) at least one enzyme composition, the enzyme component of which is selected from xylanases, glucanases and mixtures thereof in a mixture with magnesium sulfate, the magnesium sulfate fraction being about 5 to 25% by weight, based on the total weight of the dry enzyme composition;
b) at least one wheat semolina bran support, the mixing ratio of enzyme composition to support being in the range from 1:5 to 1:500; and
c) vegetable oil in a fraction of 0.1 to 1 % by weight, based on the final weight of the enzyme formulation,
the median particle size of enzyme composition and support being in the range from about 150 to 500 µm, and the xylanase fraction being 3000-30 000 TXU/g of formulation and the glucanase fraction being 2000 to 20 000 TGU/g of formulation.

10. The enzyme formulation according to claim 9, comprising an enzyme composition, the enzyme component of which is a xylanase, a glucanase, or a mixture of xylanase and glucanase; or comprising two enzyme compositions of different enzymes, the one enzyme component being a glucanase and the other a xylanase.

11. A method for producing a solid enzyme formulation according to one of the preceding claims, at least one particulate enzyme composition comprising at least one enzyme and at least one organic or inorganic salt of a monovalent or divalent cation being mixed with at least one particulate inorganic or organic support and the mixture being wetted with a hydrophobic liquid.

12. The method according to claim 11, a particulate enzyme composition comprising an enzyme in a mixture with at least one organic or inorganic salt of a monovalent or divalent cation being provided.

13. The method according to claim 11, a particulate enzyme composition comprising at least two enzymes which are different from one another being provided in a mixture with at least one organic or inorganic salt of a monovalent or divalent cation.

14. The method according to claim 11, at least two particulate enzyme compositions which are different from one another being provided, the two compositions differing in that they comprise at least one different enzyme, the enzymes in each composition being present in a mixture with at least one organic or inorganic salt of a monovalent or divalent cation.

15. The method according to one of claims 11, 12, or 13, the enzyme composition being obtained by spray drying or by spray drying and agglomeration of an enzyme-comprising liquid in which at least one organic or inorganic salt of a monovalent or divalent cation is taken up.

16. The method according to claim 11 or 14, at least two enzyme compositions of enzymes which are different from one another being obtained by spray drying or by spray drying and agglomeration of at least two different enzyme-comprising liquids in which at least one organic or inorganic salt of a monovalent or divalent cation is taken up, and
a) each of the at least two enzyme compositions being mixed with a particulate inorganic or organic support, or
b) a particulate inorganic or organic support being mixed with the at least two enzyme compositions; and
the mixture produced according to variant a) or variant b) being wetted with a hydrophobic liquid.

17. A method for producing a solid enzyme formulation comprising at least one enzyme selected from xylanases, glucanases and mixtures thereof,
a) at least one enzyme-comprising liquid being spray dried or spray dried and agglomerated to give at least one enzyme composition, the enzyme component of which being selected from xylanases, glucanases and mixtures thereof, and this enzyme component being present in the liquid in a mixture with magnesium sulfate, and the magnesium sulfate fraction being 5 to 25% by weight, based on the total weight of the dry enzyme composition;
b) the resultant enzyme composition being mixed with a particulate inorganic or organic support; and
c) the enzyme/support mixture being wetted with a hydrophobic liquid.

18. The method according to claim 17,
a) a particulate enzyme composition comprising at least one xylanase in a mixture with magnesium sulfate being provided; or
b) a particulate enzyme composition comprising at least one glucanase in a mixture with magnesium sulfate being provided; or
c) a particulate enzyme composition comprising at least one xylanase and at least one glucanase in a mixture with magnesium sulfate being provided; or
d) at least two particulate enzyme compositions which are different from one another being provided, one of the compositions comprising at least one xylanase and the other of the compositions comprising at least one glucanase, the enzymes in each composition being present in a mixture with magnesium sulfate.

19. The method according to claim 17 or 18, one or two different enzyme compositions being mixed with at least one wheat semolina bran support, the mixing ratio of enzyme composition to support being in the range from 1:5 to 1:1000.

20. The use of a dry enzyme formulation according to one of claims 1 to 10, for producing a food, food supplement or an animal feed.

21. A method for producing animal feed, food or food supplement comprising a dry enzyme formulation according to one of claims 1 to 10, comprising the production of such a dry enzyme formulation with the aid of a method according to one of claims 11 to 19, and subsequent incorporation thereof in animal feed, food or food supplement.

## Revendications

1. Formulation enzymatique solide pour incorporation dans des produits pour l'alimentation animale, des aliments ou des compléments alimentaires, comprenant un mélange
a) d'au moins une composition enzymatique particulaire, constituée d'au moins une enzyme et d'au moins un sel organique ou inorganique d'un cation monovalent ou divalent, avec
b) au moins un support particulaire inorganique ou organique, et
c) au moins un liquide hydrophobe ;
le rapport entre la granulométrie moyenne du support et celle de la composition enzymatique étant compris dans la plage de 1 à 8, le rapport de mélange de la composition enzymatique au support étant compris dans la plage de 1:1000 à 1:5 parties en poids ;
la granulométrie moyenne de la composition enzymatique et celle du support étant chacune indépendamment de l'autre comprises dans la plage de 50 à 500 µm ; et
la proportion du liquide hydrophobe étant de 0,1 à 5 % en poids par rapport au poids total de la formulation enzymatique.

2. Formulation enzymatique selon la revendication 1, comprenant une composition enzymatique particulaire, contenant une enzyme en mélange avec au moins un sel organique ou inorganique d'un cation monovalent ou divalent.

3. Formulation enzymatique selon la revendication 1, comprenant une composition enzymatique particulaire contenant au moins deux enzymes différentes l'une de l'autre, en mélange avec au moins un sel organique ou inorganique d'un cation monovalent ou divalent.

4. Formulation enzymatique selon la revendication 1, comprenant au moins deux compositions enzymatiques particulaires différentes l'une de l'autre, les deux compositions se distinguant par le fait qu'elles contiennent chacune au moins une enzyme différente, les enzymes se trouvant dans chaque composition étant présentes en mélange avec au moins un sel organique ou inorganique d'un cation monovalent ou divalent.

5. Formulation enzymatique selon l'une des revendications 2 à 4, dans laquelle la granulométrie moyenne de la composition enzymatique et celle du support sont chacune indépendamment de l'autre comprises dans la plage de 150 à 350 µm.

6. Formulation enzymatique selon l'une des revendications précédentes, dans laquelle la proportion du liquide hydrophobe est de 0,1 à 1,5 % en poids par rapport au poids total de la formulation enzymatique.

7. Formulation enzymatique selon l'une des revendications précédentes, dans laquelle la proportion des sels dans la composition enzymatique est comprise dans la plage de 1 à 30 % en poids par rapport au poids total de la composition enzymatique.

8. Formulation enzymatique selon l'une des revendications précédentes, dans laquelle la ou les enzymes sont choisies parmi les xylanases, les glucanases, les cellulases, les protéases, les kératinases, les amylases et les mélanges de celles-ci.

9. Formulation enzymatique selon l'une des revendications précédentes, la formulation comprenant un mélange
a) d'au moins une composition enzymatique dont le composant enzyme est choisi parmi les xylanases, les glucanases et les mélanges de celles-ci, en mélange avec du sulfate de magnésium, la proportion du sulfate de magnésium étant d'environ 5 à 25 % en poids par rapport au poids total de la composition enzymatique sèche ;
b) d'au moins un support de son de semoule de blé, le rapport de mélange de la composition enzymatique au support étant compris dans la plage de 1:5 à 1:500 ; et
c) d'une huile végétale, selon une proportion de 0,1 à 1 % en poids par rapport au poids final de la formulation enzymatique,
la granulométrie moyenne de la composition enzymatique et du support étant comprise dans la plage d'environ 150 à 500 µm, la proportion de la xylanase étant de 3000-30 000 TXU/g de la formulation, et la proportion de la glucanase étant de 2000 à 20 000 TGU/g de la formulation.

10. Formulation enzymatique selon la revendication 9, comprenant une composition enzymatique dont le composant enzyme est une xylanase, une glucanase ou un mélange de xylanase et de glucanase ; ou comprenant deux compositions enzymatiques de différentes enzymes, l'un des composants enzymes étant une glucanase et l'autre une xylanase.

11. Procédé de préparation d'une formulation enzymatique solide selon l'une des revendications précédentes, dans lequel on mélange à au moins un support inorganique ou organique particulaire au moins une composition enzymatique particulaire comprenant au moins une enzyme et au moins un sel organique ou inorganique d'un cation monovalent ou divalent, et on mouille le mélange avec un liquide hydrophobe.

12. Procédé selon la revendication 11, dans lequel on met à disposition une composition enzymatique particulaire contenant une enzyme en mélange avec au moins un sel organique ou inorganique d'un cation monovalent ou divalent.

13. Procédé selon la revendication 11, dans lequel on met à disposition une composition enzymatique particulaire contenant au moins deux enzymes différentes l'une de l'autre, en mélange avec au moins un sel organique ou inorganique d'un cation monovalent ou divalent.

14. Procédé selon la revendication 11, dans lequel on met à disposition au moins deux compositions enzymatiques particulaires différentes l'une de l'autre, les deux compositions se distinguant par le fait qu'elles contiennent chacune au moins une enzyme différente, les enzymes se trouvant dans chaque composition étant présentes en mélange avec au moins un sel organique ou inorganique d'un cation monovalent ou divalent.

15. Procédé selon l'une des revendications 11, 12 ou 13, dans lequel on obtient la composition enzymatique par séchage par atomisation, ou par séchage par atomisation et agglomération d'un liquide contenant des enzymes, dans lequel on a repris au moins un sel organique ou inorganique d'un cation monovalent ou divalent.

16. Procédé selon la revendication 11 ou 14, dans lequel on obtient au moins deux compositions enzymatiques d'enzymes différentes l'une de l'autre, par séchage par atomisation ou par séchage par atomisation et agglomération d'au moins deux liquides différents contenant des enzymes, dans lesquels on a repris au moins un sel organique ou inorganique d'un cation monovalent ou divalent, et dans lequel
a) on mélange chacune des au moins deux compositions enzymatiques à un support inorganique ou organique particulaire, ou
b) on mélange un support inorganique ou organique particulaire aux au moins deux compositions enzymatiques, et
on mouille avec un liquide hydrophobe le mélange obtenu selon la variante a) ou la variante b).

17. Procédé de préparation d'une formulation enzymatique solide comprenant au moins une enzyme choisie parmi les xylanases, les glucanases et les mélanges de celles-ci, dans lequel
a) on sèche par atomisation, ou on sèche par atomisation et on agglomère au moins un liquide contenant des enzymes, pour obtenir au moins une composition enzymatique, leur composant enzyme étant choisi parmi les xylanases, les glucanases et les mélanges de celles-ci, et ce composant enzyme étant contenu dans le liquide en mélange avec du sulfate de magnésium, et la proportion de sulfate de magnésium étant de 5 à 25 % en poids par rapport au poids total de la composition enzymatique sèche ;
b) on mélange la composition enzymatique ainsi obtenue à un support inorganique ou organique particulaire ; et
c) on mouille avec un liquide hydrophobe le mélange enzyme/support.

18. Procédé selon la revendication 17, dans lequel
a) on met à disposition une composition enzymatique particulaire contenant au moins une xylanase en mélange avec du sulfate de magnésium ; ou
b) on met à disposition une composition enzymatique particulaire contenant au moins une glucanase en mélange avec du sulfate de magnésium ; ou
c) on met à disposition une composition enzymatique particulaire contenant au moins une xylanase et au moins une glucanase en mélange avec du sulfate de magnésium ; ou
d) on met à disposition au moins deux compositions enzymatiques particulaires différentes l'une de l'autre, l'une des compositions contenant au moins une xylanase et l'autre composition contenant au moins une glucanase, les enzymes se trouvant dans chaque composition étant présentes en mélange avec du sulfate de magnésium.

19. Procédé selon la revendication 17 ou 18, dans lequel on mélange une ou deux compositions enzymatiques différentes à au moins un support de son de semoule de blé, le rapport de mélange de la composition enzymatique au support étant compris dans la plage de 1:5 à 1:1000.

20. Utilisation d'une formulation enzymatique sèche selon l'une des revendications 1 à 10 pour préparer un aliment, un complément alimentaire ou un produit pour l'alimentation animale.

21. Procédé de préparation d'un produit pour l'alimentation animale, d'un aliment ou d'un complément alimentaire, contenant une formulation enzymatique sèche selon l'une des revendications 1 à 10, comprenant la préparation d'une telle formulation enzymatique sèche à l'aide d'un procédé selon l'une des revendications 11 à 19, puis son incorporation dans le produit pour l'alimentation animale, l'aliment ou le complément alimentaire.
